# EUROPEAN PATENT SPECIFICATION

(11) **EP 0 539 719 B1**
(45) Date of publication and mention of the grant of the patent: **19.06.1996**
(21) Application number: 92116131.1
(22) Date of filing: 21.09.1992
(51) Int. Cl.: C07C 17/42, C07C 19/12, C11D 7/50

(54) **Stabilized 141b**
Stabilisiertes 141b
141b stabilisé

(30) Priority: 09.10.1991 US 773481
(43) Date of publication of application: 05.05.1993
(73) Proprietor: ELF ATOCHEM NORTH AMERICA, INC., Philadelphia Pennsylvania 19102-3222 (US)
(72) Inventor: Crooker, Richard McMaster, Fogelsville, Pennsylvania 18051 (US); Elsheikh, Maher Yousef, Wayne, Pennsylvania 19087 (US)
(74) Representative: Kraus, Walter, Dr.

(56) References cited:
- EP-A- 0 394 992
- EP-A- 0 421 790
- WO-A-92/10453
- US-A- 5 039 442
- CHEMICAL ABSTRACTS, vol. 111, no. 19, 6 November 1989, Columbus, Ohio, US; abstract. no. 173592h, 'Stabilization of lower chlorofluoroalkanes containing at least one hydrogen atom' page 675; & JP-A-0 150 829 (ASAHI GLAS) 27 February 1989
- R.M.Crooker et al., J. Cellular Plastics, 1989, 25(6), 609-617

## Description

### Field of the Invention

This invention relates to novel compositions containing 1,1-dichloro-1-fluoroethane ("141b") and stabilizing additives (or inhibitors) such as nitromethane, more particularly to compositions of 141b which are stabilized against decomposition in polyol premix formulations or the corresponding polyurethane or polyisocyanurate foams made therefrom.

### Background of the Invention

Polyurethane and polyisocyanurate foams are conventionally prepared by reacting an organic polyisocyanate (including diisocyanate) "A-side" component with a "B-side" polyol premix component containing organic polyol, blowing agent, surfactant, catalyst, and possibly other additives such as flame retardants_{,} antioxidants_{,} and U.V. stabilizers. These A-side and B-side components may be purchased by the end-user in separate containers and stored for later use. Since decomposition of the 141b blowing agent has been observed in the B-side premixes during storage and during the process of making the foam, 141b compositions inhibited against such decompositions would be highly desirable. For example, 141b has been observed to decompose during the foam-making process to up to about 1%, depending on the formulation and reaction conditions, of various decomposition products of which by far the predominant product is 1-chloro-1-fluoroethylene ("1131a"). Inhibition of such decomposition is desired both because of toxicity concerns and because the decomposition is accompanied by the formation of equivalent amounts of acid which in turn causes catalyst deactivation.

US-A-5 039 442 relates to stable azeotrope-like compositions consisting essentially of 1,1-dichloro-1-fluoroethane, dichloromethane and optionally alkanol which are useful in a variety of industrial cleaning applications and one of which is also useful as a blowing agent in the preparation of polyurethane foams. Accordingly, US-A-5 039 442 relates to mixtures of halocarbons.

1,1-Dichloro-1-fluoroethane (141b) was reported to be unique among halocarbons in its stability in polyols as taught by R.M. Crooker et al., "Accelerated Aging Study of HCFC 141b in Polyurethane Premix", J. Cellular Plastics (1989), 25(6), pages 609-617. This paper teaches (note particularly Tables 9 and 10 and the discussion in the paragraph bridging pages 615 and 616) that 141b is so stable to acid formation in polyol premix formulation that inhibitors such as alpha-methylstyrene are not needed. What the authors of this paper apparently did not appreciate, however, was that a toxic decomposition product, 1-chloro-1-fluoroethylene (1131a), was being formed. Thus, the present invention involves the two-fold discovery that (a) 141b was not as stable as the art thought, decomposing into 1131a and (b) that nitromethane and alpha-methyl-styrene are effective in stabilizing 141b against this decomposition.

Chem. Abs., 1989, 111(19), 173592h discloses stabilizing against acidity formation the hydrogen-containing lower chlorofluoroalkanes generally, but only exemplifies HCFC 123. This hardly suggests the present invention, especially in view of the teaching of the Crooker et al article that 141b does not need a stabilizer to prevent acidity.

### Summary of the Invention

A foam premix formulation is provided comprising a polyol, a halocarbon which consists of 1,1-dichloro-1-fluoroethane, and nitromethane as an inhibitor in an amount effective to stabilize the 1,1-dichloro-1-fluoroethane against decomposition into 1-chloro-1-fluoroethylene.

### Detailed Description of the Invention

It has now been found that 141b is stabilized against decomposition by the addition of an inhibitor selected from alpha-methylstrene or nitromethane.

The inhibitor is present in an effective amount, typically from about 0.01 to about 2% by weight, based on the weight of 141b, preferably 0.05 to 1%.

The compositions also include a polyol or a fully formulated B-side formulation containing polyol, catalyst, surfactant, and, optionally, other additives. Typical polyols are, for example, Stepanol PS 2502A, an aromatic polyester polyol sold by the Stepan Company; Terate 203, an aromatic polyester polyol sold by Hercules, Inc.; Pluracol Polyol 975, a sucrose-based polyol sold by BASF; Poly-G 71-530, a polyether polyol sold by Olin; and Quadrol, an amine-based polyol supplied by BASF. Typical catalysts include Potassium HEX-CEM, a potassium octoate sold by Mooney Chemicals; Polycat 41, an N,N,N-tri(dimethylaminopropyl)cyclohexatriazine catalyst sold by Air Products; Polycat 8, an N,N-dimethylcyclohexylamine catalyst sold by Air Products; Dabco TMR-30, a 2,4,6-tri(dimethylaminomethyl)phenol supplied by Air Products; and Dabco K-15, a potassium 2-ethylhexoate in diethylene glycol supplied by Air Products. A typical surfactant is Dow Corning 193, a silicone polymer surfactant. A typical A-side component is Mondur E-489, an aromatic diisocyanate supplied by Mobay Chemical Co., or Lupranate M20S, a polymethylenediisocyanate supplied by BASF.

The invention was illustrated by first preparing a polyurethane foam with 141b in the absence of inhibitor by stirring a formulation containing polyol (100g of Stepanol PS 2502A), 141b (25.8g), surfactant (1.51g of Dow Corning 193), catalyst (2.82g of Potassium HEX-CEM and 0.7g of Polycat 41), and diisocyanate (127.2g of Mondur E-489). The contents were poured into a box and the resulting foam was left to cool to room temperature. After curing the foam at 250°F for 20 hours, the cell gas was analyzed by crushing a sample and injecting the released gas mixture directly to a gas chromatograph. The gas was found to contain 2180 ppm of 1131a, whereas the 141b starting material contained only 10 ppm of 1131a. Other minor components in the cell gas totalled only about 440 ppm, similar to the levels found in the 141b starting material. When 0.6 weight % of each of alpha-methylstyrene and nitromethane were dissolved in 141b, foams prepared as aforesaid contained only 1390 ppm and 423 ppm of 1131a, respectively.

## Claims

1. A foam premix formulation comprising a polyol, a halo-carbon which consists of 1,1-dichloro-1-fluoroethane, and nitromethane as an inhibitor in an amount effective to stabilize the 1,1-dichloro-1-fluoroethane against decomposition into 1-chloro-1-fluoroethylene.

2. A composition as in claim 1 further comprising a polyisocyanate.

3. Use of alpha-methylstyrene (AMS) for inhibiting decomposition of 141b to 1131a in a foam premix formulation comprising a polyol and 141b.

## Patentansprüche

1. Schaum-Vormisch-Zubereitung, enthaltend ein Polyol, einen Halogenkohlenwasserstoff, welcher aus 1,1-Dichlor-1-fluorethan besteht, und Nitromethan als Inhibitor in einer Menge, die wirksam ist, um das 1,1-Dichlor-1-fluorethan gegenüber Zersetzung in 1-Chlor-1-fluorethylen zu stabilisieren.

2. Zusammensetzung nach Anspruch 1, dadurch gekennzeichnet, daß sie weiter ein Polyisocyanat enthält.

3. Verwendung von α-Methylstyrol (AMS) für die Inhibierung der Zersetzung von 141b zu 1131a in einer Schaum-Vormisch-Zubereitung, die ein Polyol und 141b enthält.

## Revendications

1. Formulation de prémélange de formulation de mousse, comprenant un polyol, un carbure halogéné qui consiste en 1,1-dichloro-1-fluoréthane, et du nitrométhane servant d'inhibiteur en une quantité efficace pour stabiliser le 1,1-dichloro-1-fluoréthane vis-à-vis de la décomposition en 1-chloro-1-fluoréthylène.

2. Composition suivant la revendication 1, comprenant en outre un polyisocyanate.

3. Utilisation d'alpha-méthylstyrène (AMS) pour inhiber la décomposition du 141b en 1131a dans une formulation de prémélange de formulation de mousse comprenant un polyol et du 141b.
